# EUROPEAN PATENT SPECIFICATION

(11) **EP 1 306 100 B1**
(45) Date of publication and mention of the grant of the patent: **27.04.2005**
(21) Application number: 01650127.2
(22) Date of filing: 23.10.2001
(51) Int. Cl.: A61M 25/00

(54) **Clip for catheter storing**
Lagerklemme für Katheter
Clip pour stockage de cathéter

(43) Date of publication of application: 02.05.2003
(73) Proprietor: Moynesign Limited, Enniscorthy, County Wexford (IE)
(72) Inventor: Jones, Andrew, The Rower, County Kilkenny (IE)
(74) Representative: O'Brien, John Augustine

(56) References cited:
- EP-A- 0 865 799
- WO-A-99/47202
- US-A- 5 429 321
- US-A- 5 954 707
- US-B1- 6 290 691

## Description

### Introduction

The invention relates to a vascular catheter and in particular to a clip for temporarily holding a catheter shaft in position adjacent to a patient when the shaft is temporarily removed from the vasculature.

Treatment of a stenosis in an artery is generally carried out transluminally using a range of treatment procedures and devices. For example, balloon angioplasty may be carried out with or without a stenting procedure. Various catheters may be introduced over a guidewire during the various treatment procedures and in most cases a number of different catheters are used at different stages. Such catheters are generally very long and cannot be easily stored in the sterile treatment environment.

Various clips have been developed in an effort to temporarily store a catheter, however clips developed to date are generally inconvenient and inefficient to use.

In international patent application published under number WO 99/47202, a catheter connector is described in which storing recesses are integrally formed with a hub. A part of a protection tube may be detachably fixed into the recesses.

There is therefore a need for an improved catheter which will overcome these problems.

### Statements of Invention

According to the invention there is provided a hub for a catheter, the hub having an integral clip for temporarily retaining a shaft of the catheter in a coiled stored configuration.

In a preferred embodiment the clip is defined by a slot provided in the hub. Preferably the slot is provided at a distal end of the hub.

The slot has an end entrance which is arranged substantially perpendicular to a longitudinal axis of the hub.

In a preferred arrangement the slot has a retainer for retaining the catheter shaft. Preferably the retainer is defined by a return leg of the slot.

In one embodiment the hub comprises at least two slots which are spaced apart around the periphery of the hub. Typically there are two slots which are provided on opposite sides of the hub.

The invention also provides a catheter comprising a hub of the invention.

### Brief Description of the Drawings

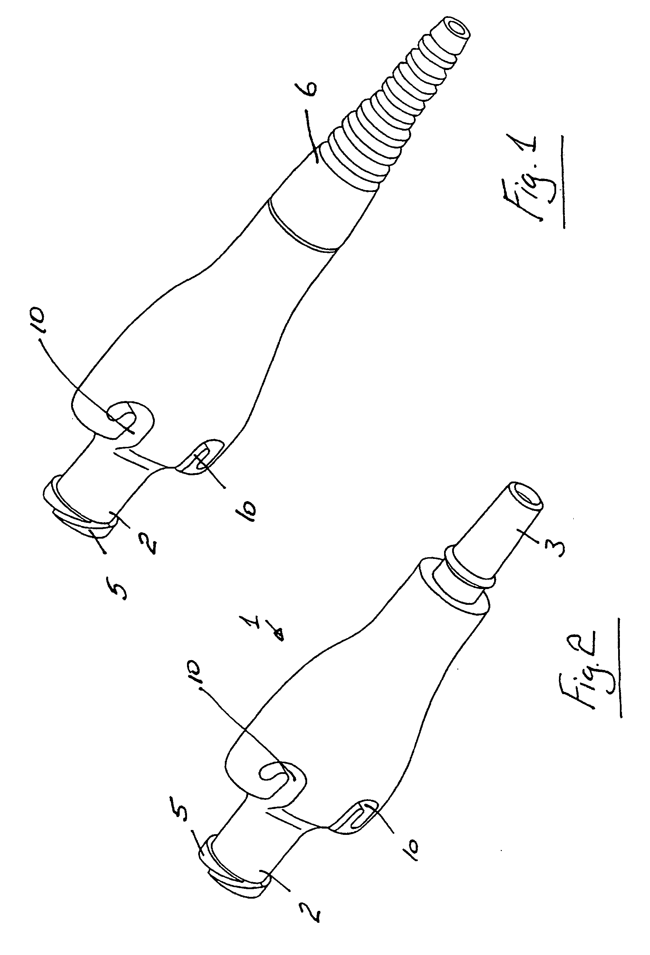

The invention will be more clearly understood from the following description thereof given by way of example only, with reference to the accompanying drawings, in which:-
- Fig. 1: is a perspective view of a catheter hub clip according to the invention;
- Fig. 2: is a perspective view of the clip with a proximal attachment removed;

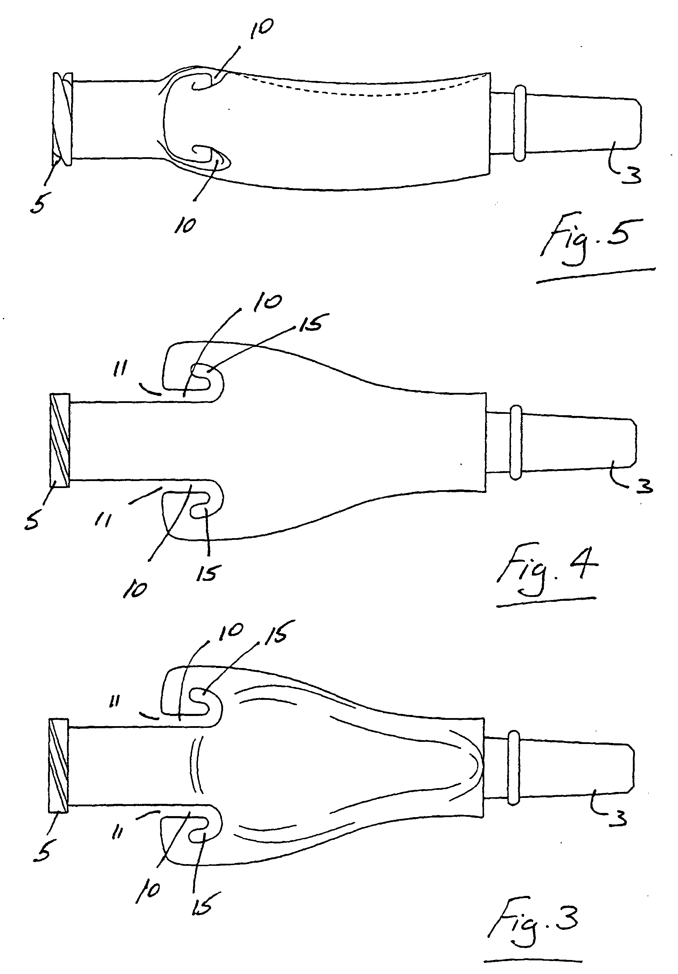

- Fig. 3: is a top plan view of the clip of Fig. 2;
- Fig. 4: is an underneath plan view of the clip;
- Fig. 5: is a side view of the clip;

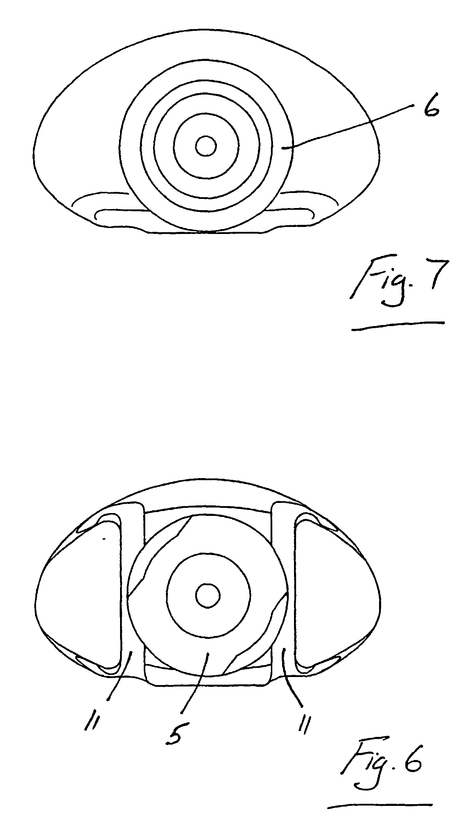

- Fig. 6: is a view of the proximal end of the clip of Fig. 1;
- Fig. 7: is a view of the distal end of the clip;

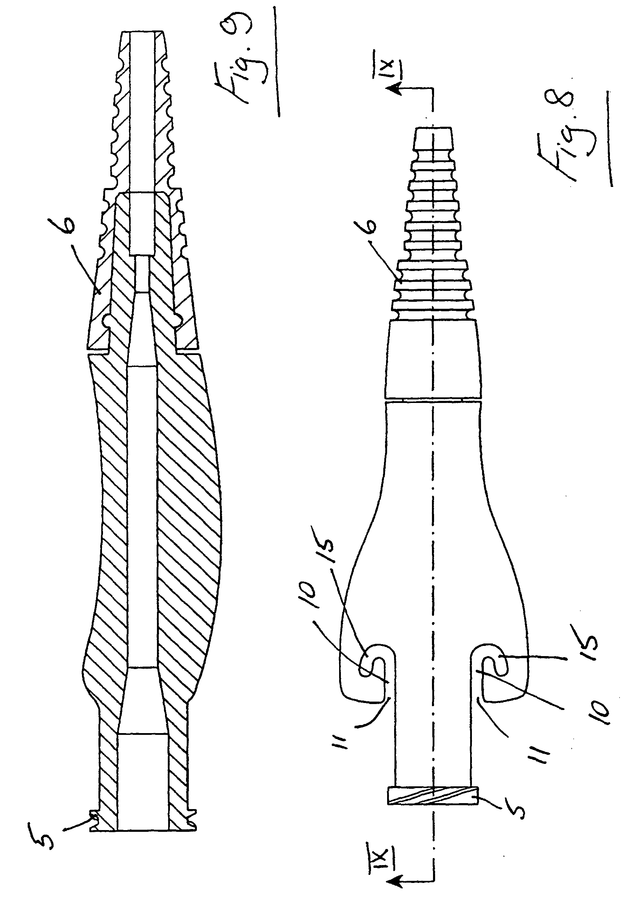

- Fig. 8: is a plan view of the clip of Fig. 1;
- Fig. 9: is a cross sectional view on the line IX - IX in Fig. 8;
- Fig. 10: is a perspective view of the clip in use;
- Fig. 11: is a side view of the clip of Fig. 10; and
- Fig. 12: is a view of the proximal end of the clip of Fig. 10.

### Detailed Description

Referring to the drawings there is illustrated a catheter hub 1 having a proximal end 2 and a distal end 3. The proximal end 2 of the hub is provided with a leuer fitting 5 for removable connection to an accessory such as a Y-adapter. The distal end 3 of the hub has a transition piece 6 for reinforcing a connection between the distal end 3 of the hub and a catheter shaft such as a hypotube 4.

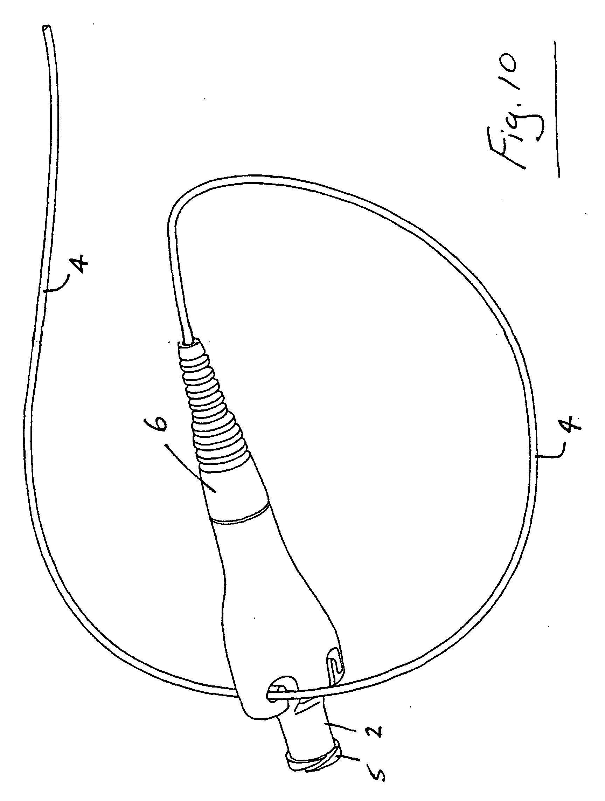

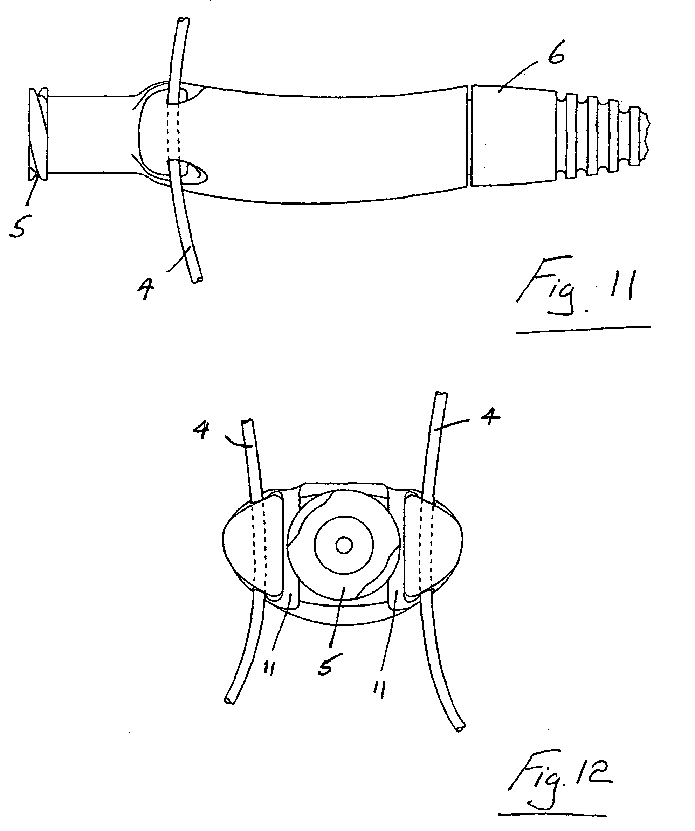

The hub 1 has an integral clip adjacent the distal end for temporarily retaining a looped hypotube (Figs. 10 to 12). The hub with the coiled hypotube can be retained in the sterile field ready for re-use by the clinician, if required.

The clip is defined in this case by two diametrically opposed slots 10. An end entrance 11 of the slots 10 is arranged substantially perpendicular to a longitudinal axis of the hub for ease of insertion of the coiled hypotube 4.

Each slot 10 has a retainer in the form of a return leg 15 in which the hypotube 4 is temporarily retained.

The hub is of relatively simple yet effective construction. Because the clip is integral with the hub, especially at the proximal end, the hypotube shaft 4 can be readily passed into, retained in and removed from the slots 10 by the clinician, in use. The clip does not interfere with the low profile of the hub and there are no moveable parts which makes the clipping and unclipping procedure relatively simple.

## Claims

1. A hub (1) for a catheter, the hub (1) defining a longitudinal axis, the hub (1) comprising a lumen extending therethrough between a proximal end (2) and a distal end (3), and the hub (1) having an integral clip for temporarily retaining a shaft of the catheter in a coiled stored configuration; the clip being defined by a slot (10) provided in the hub (1); **characterised in that**:
the slot (10) has an end entrance (11) which is arranged substantially perpendicular to the longitudinal axis of the hub (1); and
the slot (10) is spaced from the lumen in a radial direction, which is substantially perpendicular to the longitudinal axis of the hub (1) and substantially perpendicular to the axis of the end entrance (11).

2. A hub as claimed in claim 1 wherein the slot (10) is provided at the distal end (3) of the hub (1).

3. A hub as claimed in claim 1 or 2 wherein the slot (10) has a retainer for retaining the catheter shaft.

4. A hub as claimed in claim 3 wherein the retainer is defined by a return leg (15) of the slot (10).

5. A hub as claimed in any of claims 1 to 4 wherein the hub (1) comprises at least two slots (10) which are spaced apart around the periphery of the hub (1).

6. A hub as claimed in claim 5 wherein there are two slots (10) which are provided on opposite sides of the hub (1).

7. A catheter comprising a hub (1) as claimed in any preceding claim.

## Patentansprüche

1. Ansatzstück (1) für einen Katheter, wobei das Ansatzstück (1) eine Längsachse begrenzt, das Ansatzstück (1) einen durch dasselbe verlaufenden Hohlraum zwischen einem proximalen Ende (2) und einem distalen Ende (3) aufweist, und das Ansatzstück (1) eine integrierte Klemme zum zeitweiligen Festhalten eines Schafts des Katheters in einer spiralförmig gelagerten Konfiguration aufweist, wobei die Klemme durch einen in dem Ansatzstück (1) vorgesehenen Schlitz (10) definiert wird, **dadurch gekennzeichnet, dass**
der Schlitz (10) einen Endeingang (11) umfasst, der im wesentlichen senkrecht zur Längsachse des Ansatzstücks (1) angeordnet ist; und
der Schlitz (10) von dem Hohlraum in einer radialen Richtung beabstandet ist, die im wesentlichen senkrecht zur Längsachse des Ansatzstücks (1) und im wesentlichen senkrecht zur Achse des Endeingangs (11) ist.

2. Ansatzstück nach Anspruch 1, wobei der Schlitz (10) am distalen Ende (3) des Ansatzstücks (1) vorgesehen ist.

3. Ansatzstück nach Anspruch 1 oder 2, wobei der Schlitz (10) einen Halter zum Festhalten des Katheterschafts aufweist.

4. Ansatzstück nach Anspruch 3, wobei der Halter durch ein Rückstellbein (15) des Schlitzes (10) definiert wird.

5. Ansatzstück nach einem der Ansprüche 1 bis 4, wobei das Ansatzstück (1) mindestens zwei Schlitze (10) aufweist, die um den Umfang des Ansatzstücks (1) herum voneinander beabstandet sind.

6. Ansatzstück nach Anspruch 5, wobei zwei Schlitze (10) vorhanden sind, die an entgegengesetzten Seiten des Ansatzstücks (1) vorgesehen sind.

7. Katheter mit einem Ansatzstück (1) nach einem vorhergehenden Anspruch.

## Revendications

1. Embase (1) pour cathéter, l'embase (1) définissant un axe longitudinal, l'embase (1) comprenant une lumière se prolongeant au travers de celle-ci entre une extrémité proximale (2) et une extrémité distale (3), et l'embase (1) ayant un clip intégral destiné à retenir temporairement une tige du cathéter en une configuration rangée en boucle ; le clip étant défini par une encoche (10) prévue dans l'embase (1) ;
**caractérisée en ce que** :
l'encoche (10) présente une entrée d'extrémité (11) qui est disposée dans une large mesure de manière perpendiculaire par rapport à l'axe longitudinal de l'embase (1) ; et
l'encoche (10) est espacée de la lumière dans une direction radiale, qui est dans une large mesure perpendiculaire par rapport à l'axe longitudinal de l'embase (1) et dans une large mesure perpendiculaire par rapport à l'axe de l'entrée d'extrémité (11).

2. Embase selon la revendication 1, dans laquelle l'encoche (10) est prévue à l'extrémité distale (3) de l'embase (1).

3. Embase selon la revendication 1 ou la revendication 2, dans laquelle l'encoche (10) est munie d'un dispositif de retenue destiné à retenir la tige du cathéter.

4. Embase selon la revendication 3, dans laquelle le dispositif de retenue est défini par un retour (15) de l'encoche (10).

5. Embase selon l'une quelconque des revendications 1 à 4, dans laquelle l'embase (1) comprend au moins deux encoches (10) qui sont espacées l'une de l'autre sur la périphérie de l'embase (1).

6. Embase selon la revendication 5, dans laquelle il y a deux encoches (10) qui sont prévues sur les côtés opposés de l'embase (1).

7. Cathéter comprenant une embase (1) selon l'une quelconque des revendications précédentes.
